**Europäisches Patentamt**

(19) **European Patent Office**

**Office européen des brevets**

(11) Veröffentlichungsnummer: **0 093 869**

**B1**

(12) # EUROPÄISCHE PATENTSCHRIFT

(45) Veröffentlichungstag der Patentschrift: 29.04.87

(51) Int. Cl.⁴: **A 61 F 2/30**

(21) Anmeldenummer: 83102973.1

(22) Anmeldetag: 25.03.83

(54) Individuell gestaltete Gelenkendoprothesen.

(30) Priorität: 10.04.82 DE 3213434

(43) Veröffentlichungstag der Anmeldung:
16.11.83 Patentblatt 83/46

(45) Bekanntmachung des Hinweises auf die Patenterteilung:
29.04.87 Patentblatt 87/18

(84) Benannte Vertragsstaaten:
AT BE CH DE FR GB IT LI NL SE

(56) Entgegenhaltungen:
EP-A-0 025 911
EP-A-0 040 165
EP-A-0 054 785
DE-A-3 123 939

BIOMEDIZINISCHE TECHNIK, Band 26, Heft 1-2,
Jänner-Februar 1981 C.H. Siemens "Systematik der
Entwicklung einer Endoprothese von der Planung
bis zur Implantation, dargestellt am Beispiel der
Sprunggelenk-Endoprothese. Modell BME" Seiten
19-27

(73) Patentinhaber: Aldinger, Günther Dr. med.,
Engelfriedshalde 13, D-7400 Tübingen (DE)

(72) Erfinder: Aldinger, Günther Dr. med.,
Engelfriedshalde 13, D-7400 Tübingen (DE)

(74) Vertreter: Möbus, Rudolf, Dipl.- Ing.,
Hindenburgstrasse 65, D-7410 Reutlingen (DE)

**Beschreibung**

Die Erfindung betrifft eine Gelenkendoprothese mit einem zum Einsetzen in einen von einer Knochenöffnung aus geschaffenen Knochenhohlraum bestimmten, in einzelne Abschnitte unterschiedlichen Querschnitte unterteilten, einer geradlinigen Bereich aufweisenden Verankerungsschaft.

Bei Prothesen, insbesondere Endoprothesen, hat sich die dauerhafte Verankerung der Prothesen als ein Hauptproblem herausgestellt, dies gilt sowohl für totale Endoprothesen (z. B. nach Hüftkopf-Hals-Resektion) als auch für Gelenkflächenersatz-Endoprothesen (z. B. Hüftkopfkappen-, Knieschlitten-Endoprothesen) sowie deren Kombinationen (z. B. Oberschenkelanteil einer Knietotalendoprothese). Der Variation der durch die Prothesen oder Implantate zu ersetzenden natürlichen Körperteile in Größe, Formgebung, Struktur, Winkelstellung, Längen- und Breitengestaltung usw. steht eine relativ geringe Anzahl konfektionierter Prothesen oder Prothesenteile, Stütz- oder Volumenersatzteile aus verschiedenen Werkstoffen gegenüber. Dies bedeutet, daß der Chirurg in den meisten Fällen gezwungen ist, eine Anpassung der umliegenden oder zur Verankerung der Endoprothese herangezogenen Knochen des Patienten an das vorgegebene Prothesenmodell vorzunehmen. In den überwiegenden Fällen bedeutet dies, insbesondere bei oberflächenverankerten Endoprothesen, die Entfernung von Knochensubstanz zur Anpassung oder Einpassung der konfektionierten Prothese, wobei zwangsläufig an der Protheseneinsatz- oder -ansatzstelle wertvolle, an dort auftretende Druck-, Zug- und Torsionskräfte angepaßte Knochenstruktur beeinträchtigt wird. Bei der konventionellen, schaftimplantierten Endoprothese (z. B. einer Hüftendoprothese), muß ein relativ schmächtiger Prothesenstiel in einem relativ weiten natürlichen Knochenschaft verankert werden. Läßt man hier Spongiosa stehen, ist deren Balken- oder Maschenstruktur trotzdem gestört, so daß sie ihre ursprüngliche Stützfunktion nicht mehr erfüllen kann. Bei der zementverankerten konventionellen Endoprothese wird die Spongiosa weitgehend entfernt und der dann zwischen Prothese und Compacta des Knochens verbleibende Freiraum wird durch Einbringen eines Füllmaterials, z. B. Knochenzement, ausgefüllt. Dieses Verfahren hat jedoch sowohl bei der konventionellen totalen Endoprothese als auch bei der Oberflächenersatzendoprothese und deren Kombination in Abhängigkeit von der Implantationstechnik, der Schichtdicke des Zementes, der Elastizitätskoeffizienten und des Implantationszeitraumes langfristig enttäuscht (siehe z. B. Harris W. H. "Bony Ingrowth Fixation of Acetabular Components for Canine Total Hip Replacement", 27. Ann. Meeting Orth. Res. Soc., 1981, Vol. 6, Seite 74).

Es wurden zwar bereits zementfreie Implantationsmöglichkeiten gesucht, und es wurde versucht, durch großvolumige Prothesenschäfte, längere Prothesenschäfte und ein besseres Prothesenschaft- und Prothesendesign mit weniger Zement bzw. dünneren Zementschichten auszukommen. Diese Versuche müssen aber praktisch als gescheitert angesehen werden, da es einfach nicht möglich ist, mit konfektionierten, also vorgefertigten Prothesen mit einer realistischen Anzahl von unterschiedlichen Größen die Variationsbreite menschlicher Gelenke und Gelenkanteile zu erfassen, selbst wenn Endoprothesen mit zur Anpassung verstellbaren Teilen verwendet werden.

Das erwähnte Hauptproblem der mit der Zeit auftretenden Lockerung der Endoprothesen muß in der mangelnden Paßgenauigkeit der an- oder einzupassenden, industriell gefertigten Prothesen bei den praktisch unendlichen Variationsmöglichkeiten der Gelenkanteile gesehen werden. Dies gilt bei Gelenken sowohl für den totalen als auch für den Gelenkflächenersatz oder deren Kombinationen. Schon beim Oberflächenersatz müssen z. T. wichtige kraftaufnehmende und kraftverteilende Knochenstrukturen (harte Knochenrinde, Corticalis) oder gelenknahes Knochengeflecht (Spongiosa) zur Kraftaufnahme und Verteilung beispielsweise in das corticale compacte Knochenschaftrohr entfernt oder bestehende Inkongruenzen durch Füllmaterial ausgeglichen werden.

Man hat nun versucht, die Verankerungsprobleme mit einer Endoprothese zu lösen, deren Verankerungsteil in einzelne Abschnitte unterteilt ist, die über elastische Schichten miteinander verbunden sind, die einen runden Querschnitt haben und gegeneinander so versetzt angeordnet sind, daß aufeinanderfolgende Abschnitte an gegeneinander versetzten Umfangsstellen gegen die Wandung einer Knochenhöhle zwecks Abstützung zur Anlage kommen (DE-A- 31 23 939). Eine solche Endoprothese hat den Nachteil, daß die runden Schaftabschnitte praktisch immer nur an einer einzigen Stelle zur Anlage gegen den Knochen gelangen. Auch entstehen bei einer solchen Schaftgestaltung zwangsläufig Hinterschneidungen zwischen den einzelnen scheibenförmigen Schaftabschnitten. Die Abstützung ist zu gering, was einen Knochenschwund an den nicht belasteten Knochenwandungsstellen begünstigt. In die Hinterschneidungsteile wächst weiches Knochengeflecht (Spongiosa) ein, das nach einer späteren Schrumpfung des Knoches mit einem Zurückweichen der Knochenrinde (Corticalis) nicht imstande ist, die Prothese zu stützen, die dann unter Absicherung des Knochengeflechtes tiefer gedrückt wird.

Der Erfindung liegt die Aufgabe zugrunde, eine Gelenkendoprothese der eingangs genannten Art individuell anatomisch zu gestalten und dadurch eine optimale und dauerhafte Verankerung sowie eine optimale Biomechanik der Endoprothese zu ermöglichen.

Die gestellte Aufgabe wird erfindungsgemäß dadurch gelöst, daß der Verankerungsschaft mindestens in den Schaftbereichen, in welchen eine Abstützung des Verankerungsschaftes am Knochen erfolgt, in einzelne, jeweils rundum verlaufende Stufen gegliedert ist, die mindestens in dem geradlinigen Bereich des Verankerungsschaftes von seinem gelenkfernen Ende aus ohne Hinterschneidungen aufeinanderfolgen und einen sich trichterartig erweiternden Verankerungsschaft ergeben und deren Kontur mindestens über den Umfangsbereich, in welchem eine Abstützung der Stufen am Knochen erfolgt, individuell an denjenigen Querschnitt des Knochenhohlraumes angepaßt ist, der bei eingesetzter Endoprothese an der Stelle der einzelnen Stufen vorliegt, wobei die individuelle Anpassung durch folgende Schritte bewirkt worden ist:

a) Herstellung mehrerer maßstabsgerechter, nach den Prothesenschaftstufen ausgewählter Bereichsbilder von dem mit der Endoprothese zu versehenden Knochen mittels der Computer-Tomographie oder Kernspin-Resonanz-Tomographie;

b) Erfassung des von der Endoprothese einzunehmenden Bereiches in den einzelnen Abbildungen und Speicherung dieser Bereichsdaten;

c) räumliche Kombination der Bildbereiche anhand der gespeicherten Bereichsdaten;

d) Überprüfung und Modifikation der Werte der räumlichen Kombination nach vorgegebenen Kriterien, wie Querschnittsverjüngung in einer vorgegebenen Richtung, Oberflächenreliefbildung, Hohlraumbildung, Belastbarkeit, Materialkonstanten;

e) Herstellung der Endoprothese anhand der überprüften und modifizierten gespeicherten Kombinationswerte.

Zwar ist es bereits bekannt, bei der Herstellung von Zahnersatz durch eine berührungslose Abtastung von Zahnlücken und Zahnkavitäten ein räumliches Bild von der Einsätz- oder Ansatzstelle eines Zahnersatzteiles zu erhalten (EP-A- 0 040 165). Dabei soll die Abtastung durch Holographie oder mittels Laserstrahlen durchgeführt werden, und aus den Meßwerten soll dann die Form des Zahnersatzteiles oder einer anderen Prothese bestimmt werden. Diese optische Abtastung ist nur bei außenliegenden Prothesen möglich. Zwar ist es ebenfalls bekannt, mit Hilfe der Computer- oder Kernspin-Tomographie auch nicht freiliegende Knochen zu erfassen und mit Computerhilfe aus den Meßdaten eine Nachbildung dieses Knochens zu erhalten. Bei der Gelenkendoprothese gemäß der Erfindung geht es aber nicht einfach um den Ersatz eines Knochens oder die Schaffung einer in ihrer Formgestaltung relativ großen Spielraum lassenden Zahnprothese, sondern darum, das Problem der Verankerung einer Endoprothese auf oder in einem Knochen zu lösen, ohne daß ein nachträgliches Lockern der Endoprothese befürchtet werden muß. Die Erfindung geht also über die Schaffung einer maßstabsgerechten Nachbildung eines Körperteiles über einzelne auf Schichtplatten übertragene Bereichsbilder hinaus und führt zu einer individuell gestalteten Endoprothese, die in der Regel nicht nur ein genaues Abbild eines zu ersetzenden Knochens sein soll, sondern eine in einem insbesondere durch die Computer-Tomographie erfaßten Knochen zu verankernde Endoprothese sein muß. Übereinstimmung mit dem Gegenstand der EP-A- 0 040 165 besteht nur in der übergeordneten allgemeinen Aufgabenstellung, einem Menschen eine an einer Stelle seines Körpers passende Prothese vorzubereiten, ohne daß hierzu am Menschen unter Eingriffen oder Schmerzeinwirkung Maß genommen werden muß.

Die erfindungsgemäß ausgebildeten Gelenkendoprothesen sind genau an den einzelnen Anwendungsfall angepaßt, so daß ein anatomisch optimaler Sitz der Prothese in einem als Verankerung dienenden Knochen und eine weitgehende Wahrung der Struktur des tragenden Knochens erreicht werden. Damit ist die Voraussetzung für eine bleibend wirksame Verankerung der Prothese gegeben. Durch die den individuellen anatomischen Gegebenheiten angepaßte Paßform der Endoprothese kann in den meisten Fällen die Anwendung von Knochenzement auf ein Minimum beschränkt bleiben oder völlig überflüssig werden. Bei Knochenschwund kann sich die Endoprothese nachsetzen. Vor allem aber können mit der Individualprothese individuelle biomechanische und auch therapeutische Gesichtspunkte besser als bisher Berücksichtigung finden.

Bei einer erfindungsgemäß ausgebildeten Endoprothese können die Kanten der Stufen des Verankerungsschaftes gebrochen sein und vorteilhafterweise in den bevorzugten Abstützbereichen und/oder den gekrümmten Bereichen des Verankerungsschaftes eine kleinere Höhe als in den übrigen Schaftbereichen aufweisen.

Bei erfindungsgemäß ausgebildeten Hüftendoprothesen kann durch das gewählte Herstellverfahren der Verankerungsschaft auf eine sogenannte Antetorsionsebene, die nach dem Antetorsionswinkel des Gelenkkopfhalsanteils des zu ersetzenden Knochens gewählt wird, ausgerichtet aufgebaut sein. Dabei kann die sogenannte Antetorsionsebene gekrümmt verlaufen und/oder eine begrenzte Verwindung aufweisen. Der größere Herstellungsaufwand bei erfindungsgemäß ausgebildeten Endoprothesen wird durch die erzielbare höhere Passungsgüte, der damit verbundenen höheren Erfolgsquote und vor allem der erzielbaren längeren Funktionswirkung der Endoprothesen mehr als wettgemacht. Auch bringt das bei der Herstellung der Endoprothesen angewandte Verfahren den weiteren Vorteil, daß es weitgehend automatisch mit Computerhilfe und einem entsprechend erhöhten Sicherheitsfaktor ablaufen kann. Bei der Erfassung des von einer Prothese einzunehmenden Bereiches der Schichtbilder kann auch ein entsprechender Spezialist tätig sein, indem er durch eine Konturenbildung mittels eines Deckstiftes oder eines elektronischen Schreibstiftes oder durch eine Flächenabdeckung den Querschnittsverlauf der Prothese weitgehend vorgibt, doch wird in der Regel, da die Schichtbilder klare Konturen ergeben, diese Tätigkeit von automatisch arbeitenden Abtasteinrichtungen übernommen werden. Der von der Endoprothese einzunehmende Bereich der Schichtbilder kann automatisch durch Abtasten mittels eines Licht- oder Elektronenstrahles erfaßt oder vom Computer ausgefiltert werden, und die Speicherung aller Erfassungsdaten der einzelnen Verfahrensschritte kann zweckmäßig in einem Computer erfolgen, der aus den gespeicherten Daten automatisch eine räumliche Kombination der Bereiche der Schichtbilder durchführen und der nach einem vorgegebenen Prüfprogramm, z. B. einer Kollisionsmessung, auch Korrekturen und Modifikationen der erfaßten und gespeicherten Werte durchführen kann. So kann der Computer überwachen, daß eine hinterschneidungsfreie Aufeinanderfolge der einzelnen Stufen des Verankerungsschaftes eingehalten wird.

Aus welchem Material die Prothese gebildet ist, ist unwesentlich. Hiervon wird lediglich die Herstellung der Prothese anhand der ermittelten Kombinationswerte beeinflußt. So kann beispielsweise die Endoprothese mit ihrem Verankerungsschaft aus einem gegossenen, geschmiedeten oder gepreßten Rohling mittels materialabtragender Werkzeuge geformt sein. Der Rohling kann dabei an beiden Enden nachträglich entfernbare, Einspann- oder Zentrierflächen bildende Ansatzkörper aufweisen. Die Endoprothese könnte aber auch anhand eines geschaffenen maßstabsgerechten dreidimensionalen Modelles gefertigt werden.

Weitere bevorzugte Merkmale sind in den Ansprüchen 2, 7 und 10 angegeben.

Individuell gestaltete Gelenkendoprothesen gemäß der Erfindung sind mindestens in ihren Abstützbereichen an den tragenden Knochen angepaßt.

Das Herstellverfahren läßt auch die Berücksichtigung einer osteogenetisch wirksamen oder auch stoßdämpfenden Beschichtung des Endoprothesenkörpers zu. Beim Einsatz von Computern bei der räumlichen Kombination erfaßter Bereichsdaten der einzelnen Schichtbilder ist es mit Hilfe vorgegebener Steuerprogramme auch möglich, bei der räumlichen Kombination der erfaßten Bereichsdaten Hohlräume in der Endoprothese, Aussparungen zwischen bestimmten Anlagebereichen und selbst eine Schraubengangoberfläche eines Endoprothesen-Verankerungsteiles zu erzielen. Der Einsatz von Computern hat den besonderen Vorteil, daß eine Überprüfung der Belastbarkeit einer erfindungsgemäß individuell angepaßten Endoprothese nicht nachträglich an der gefertigten Endoprothese oder an einem Endoprothesenmodell geprüft werden muß, sondern bereits bei der räumlichen Kombination der erfaßten Bereichsdaten vom Computer anhand eines entsprechenden Prüfprogrammes (z. B. Finite-Element-Programmes) vorgenommen werden kann.

Nachfolgend werden zwei Ausführungsbeispiele von Endoprothesen, die erfindungsgemäß individuell nach den gewonnenen und gespeicherten Bereichsdaten mit Hilfe materialabtragender Werkzeuge gefertigt worden sind, anhand der beiliegenden Zeichnung näher beschrieben.

Im einzelnen zeigen:

Fig. 1 einen Längsschnitt in der sogenannten Antetorsionsebene durch einen mit einer Hüftgelenk-Endoprothese besetzten Oberschenkelknochen;

Fig. 2 + 3 der in Fig. 1 bezeichnete Bildausschnitt II, III in vergrößertem Maßstab bei unterschiedlich gestalteter Endoprothese;

Fig. 4 einen gegenüber der Schnittebene nach Fig. 1 um 90° gedrehten Längsschnitt senkrecht zur Antetorsionsebene durch den endoprothesenbesetzten Oberschenkelknochen;

Fig. 5 - 7 Querschnitte durch den Oberschenkelknochen entlang der Schnittlinien V - V, VI - VI und VII - VII in Fig. 1;

Fig. 8 eine Einzelansicht eines Verankerungsschaftes einer Hüftgelenk-Endoprothese mit der Antetorsionsebene;

Fig. 9 eine Einzeldarstellung einer bei einer Endoprothese möglichen gekrümmten Antetorsionsebene;

Fig. 10 eine Darstellung einer bei einer Hüftgelenk-Endoprothese auch möglichen gekrümmten und verwundenen Antetorsionsebene;

Fig. 11 einen Querschnitt durch das abgeschnittene und mit einer Endoprothese besetzte Ende eines Schienbeinknochens;

Fig. 12 eine Ansicht des Schienbeinknochenendes in Richtung des Pfeiles XII in Fig. 11.

Fig. 13 eine Seitenansicht eines Prothesenrohlings für die Herstellung einer Endoprothese nach Fig. 1 - 4.

Fig. 14 in der Fig. 6 entsprechender Schnitt durch den Oberschenkelknochen mit einem Auswertungsraster.

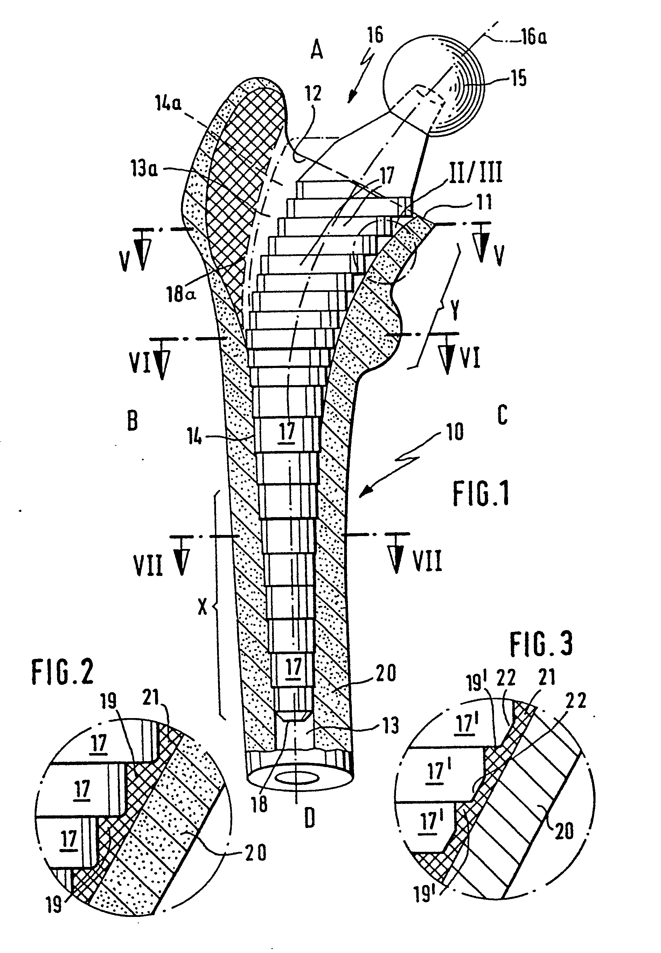

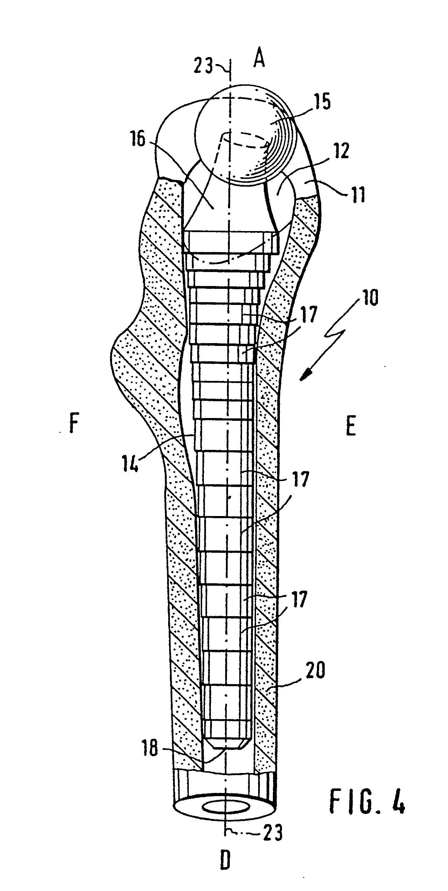

In den jeweils um 90° gegeneinander versetzten Schnittdarstellungen der Fig. 1 und 4 sind die Seiten des dargestellten Oberschenkelknochens 10 durch Großbuchstaben gekennzeichnet. A bedeutet oben, D unten, B bezeichnet die Außenseite, C die Innenseite, E die Vorderseite und F die hintere Seite des Oberschenkelknochens 10. Der Kopf des Oberschenkelknochens 10 ist entlang einer Ebene 11 abgeschnitten. Von der dadurch geschaffenen Knochenöffnung 12 aus ist durch Ausschaben von Spongiosa ein Knochenhohlraum 13 geschaffen, in welchen der Verankerungsschaft 14 des einen Gelenkkopf 15 tragenden Teiles einer Hüftgelenk-Endoprothese 16 eingesetzt ist. Der Verankerungsschaft, dessen Seele 16 a in Fig. 1 mit einer strichpunktierten Linie angedeutet ist, ist in einzelne, jeweils rundum verlaufende Stufen 17 gegliedert. In dem weitgehend geradlinig verlaufenden vorderen und unteren Endabschnitt des Verankerungsschaftes 14 weisen die Stufen 17 eine relativ große Stufenhöhe auf und folgen von der Schaftspitze 18 aus ohne Hinterschneidungen aufeinander, so daß sich ein trichterartig erweiternder Schaft ergibt. Im anschließenden, zum Schenkelhals hin gekrümmten, sich stärker trichterförmig erweiternden Längenbereich des Verankerungsschaftes 14 sind die aufeinanderfolgenden Stufen 17 mit einer kleineren Stufenhöhe ausgebildet.

Die Konturen der einzelnen Stufen 17 sind mindestens in den in Fig. 1 bezeichneten Hauptabstützungsbereichen X und Y des Verankerungsschaftes 14 individuell an den Querschnitt des geschaffenen Knochenhohlraumes 13 angepaßt, der an der Stelle der einzelnen Stufen 17 des eingesetzten Verankerungsschaftes 14 vorliegt. Im unteren Abstützungsbereich X kann die Konturenanpassung der Stufen 17 über den ganzen Umfang des Schaftumfanges 14 vorhanden sein. Im oberen Abstützungsbereich Y ist die Konturenanpassung an den Knochenhohlraum 13 jedoch überwiegend auf den konkaven Krümmungsbereich des Verankerungsschaftes 14 begrenzt, in welchem die Abstützung auch weitgehend stattfindet. Im konvexen Krümmungsbereich sind dagegen die Stufen 17 auch mit Hinterschneidungen in Richtung von der Schaftspitze 18 zum Gelenkkopf 13 gesehen angeordnet oder ganz aufgehoben und liegen beim Ausführungsbeispiel aus Gründen der guten Einsetzbarkeit des Schaftes nicht an der Begrenzungswandung 18 a des geschaffenen Knochenhohlraumes 13 an. Der dort für das Einführen des Verankerungsschaftes 14 erforderliche, nach dem Einsetzen der Endoprothese aber freibleibende Bereich 13 a (Fig. 1) wird anschließend mit Spongiosa ausgefüllt, könnte aber auch durch einen verbreiterten Rückenbereich 14 a des Prothesenschaftes ausgefüllt werden.

Wie die vergrößerten Teilschnittbilder der Fig. 2 und 3 zeigen, sind die zwischen einzelnen Stufen 17 befindlichen Randräume 19 des Verankerungsschaftes 14 mit Spongiosa gefüllt, da auf der Compacta 20 des Oberschenkelknochens 10 im Abstützbereich Y eine Übergangsschicht 21 von Spongiosa verbleibt. In Fig. 3 ist ein Verankerungsschaft mit Stufen 17' dargestellt, bei welchem die Stufenkanten 22 gebrochen sind, wodurch sich kleinere Räume 19' zwischen den einzelnen Stufen 17' ergeben.

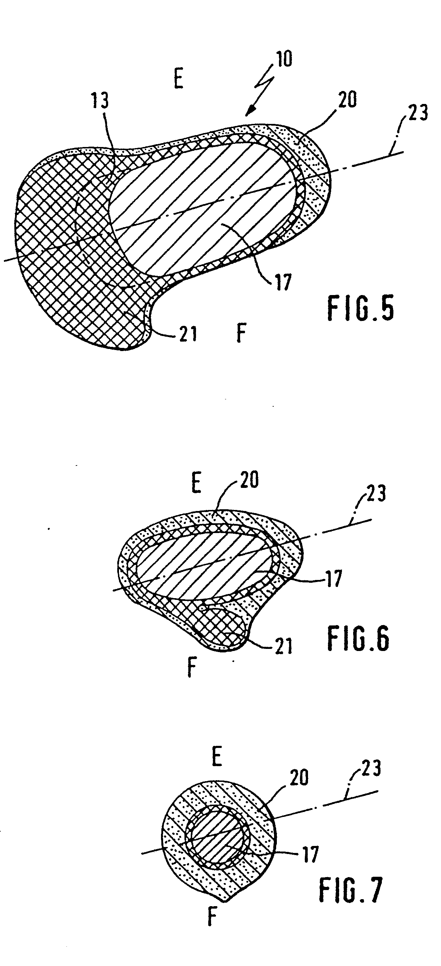

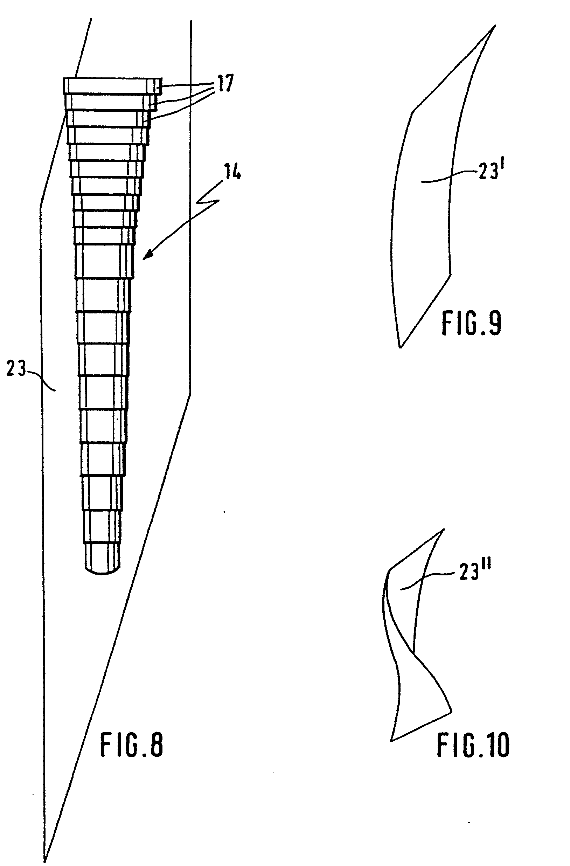

Die Querschnitte der Fig. 5 bis 7 durch den Oberschenkelknochen 10 zeigen die individuelle Anpassung der Konturen der an den einzelnen Schnittstellen befindlichen Stufen 17 des Verankerungsschaftes an den Querschnitt des im gelenknahen Bereich durch Ausschaben von Spongiosa geschaffenen Knochenhohlraumes 13. Die Spongiosabereiche 21 sind in den Schnittbildern durch eine Kreuzschraffur angedeutet und von den Compactabereichen 20 des Oberschenkelknochens 10 unterschieden. In den Schnittfiguren 3 bis 7 ist jeweils mit einer Linie 23 der Verlauf der sogenannten Antetorsionsebene angedeutet, die nach dem Antetorsionswinkel des Oberschenkel-Gelenkkopfes ausgewählt ist und nach welcher der Verankerungsschaft 14 ausgerichtet ist. Der Verlauf der Antetorsionsebene ist aus der in Fig. 4 strichpunktiert eingetragenen Linie 23 erkennbar. Eine beim Verankerungsschaft 14 auch mögliche gekrümmte Antetorsionsebene 23' ist schematisch in Fig. 9 dargestellt. In der Ansicht nach Fig. 8, die dem Schnitt nach Fig. 4 entspricht, ist die Antetorsionsebene 23 als ungekrümmte Ebene eingezeichnet. Fig. 10 zeigt schematisch eine Antetorsionsebene 23'', die sowohl gekrümmt ist als auch eine Verwindung aufweist und nach welcher ebenfalls Verankerungsteile von Hüftgelenk-Endoprothesen geformt werden können.

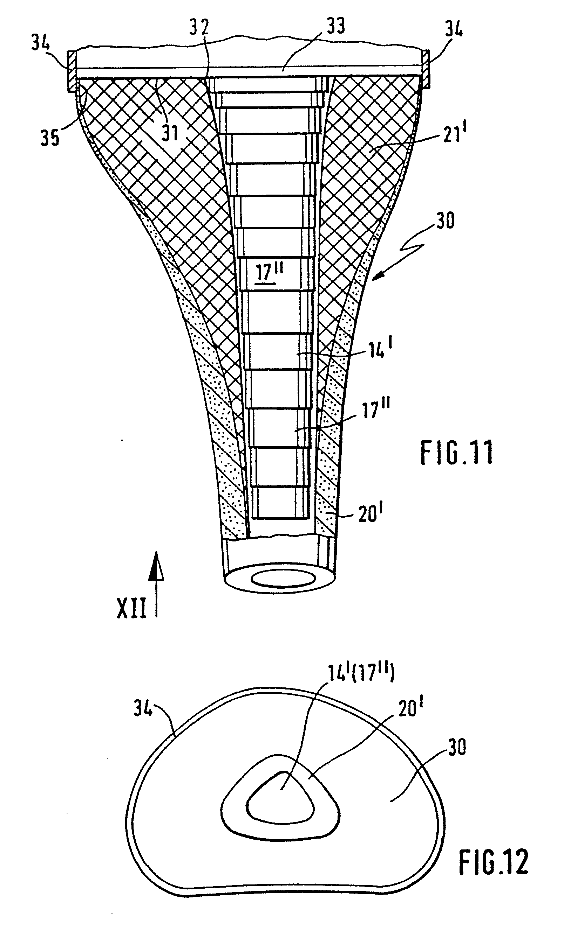

Fig. 11 zeigt den gestuften, ungekrümmt verlaufenden Verankerungsschaft 14' eines Teiles einer Kniegelenk-Endoprothese, der in einem Schienbeinknochen 30 verankert ist. Der Kopf des Schienbeinknochens 30 ist unter Bildung einer ebenen Schnittfläche 31 abgetrennt. Auch dort ist ein Knochenhohlraum zur Aufnahme des gestuften und sich in Richtung auf die Knochenöffnung 32 trichterartig erweiternden Verankerungsschaftes 14' im Kopfteil des Schienbeinknochens 30 aus der Spongiosa 21' herausgearbeitet. Im verjüngten Schaftbereich des Schienbeinknochens 30 sind die in ihrer Kontur individuell an den jeweiligen Querschnitt des Knochenhohlraumes angepaßten Stufen 17'' des Verankerungsschaftes 14' direkt an der Compacta 20' anliegend, wie Fig. 12 erkennen läßt. Der Verankerungsschaft 14' endet in einer Platte 33, welche die gesamte Schnittfläche 31 des Schienbeinknochens 30 abdeckt und die in einem Randflansch 34 endet, welcher genau dem Außenrand der Schnittfläche 31 des Knochens folgt und mit einem nach unten gerichteten Flanschteil den Knochenrand 35 umschließt. Die Platte 33 mit ihrem Flanschrand 34 bildet somit neben ihrer hier nicht näher interessierenden Gelenkfunktion eine Sicherung der Endoprothese mit ihrem gestuften Verankerungsschaft 14' gegen Verdrehen.

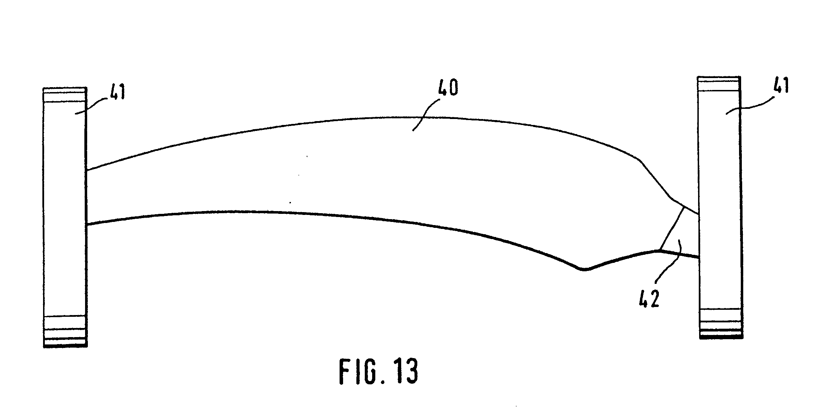

Fig. 13 zeigt einen z. B. aus einer Stahllegierung vorgeschmiedeten Endoprothesenrohling 40, an dessen beide Enden parallel zueinander ausgerichtete Scheibenkörper 41 zum Halten und zum Zentrieren des Prothesenrohlings 40 in einer Maschine mit materialabtragenden Werkzeugen angeformt oder angeschweißt sind. Der Rohling 40 ist zur Herstellung einer Hüftgelenk-Endoprothese 16 mit dem gestuften Verankerungsschaft 14 bestimmt. Nach der Fertigstellung des gestuften Schaftes werden die beiden Scheibenkörper 41 abgetrennt.

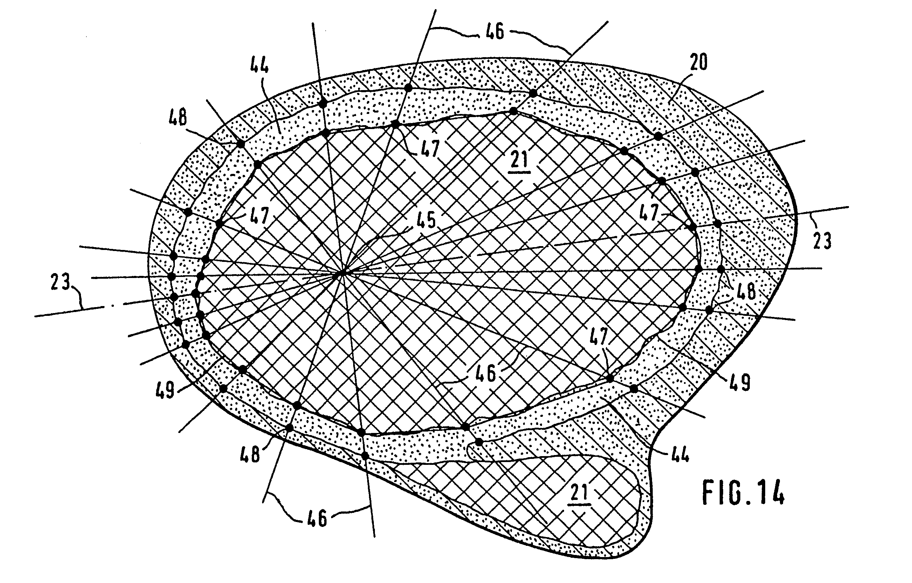

Die Erfassung des von der Prothese einzunehmenden Bereiches in den ermittelten einzelnen Bereichsbildern eines Knochens oder anderen Körperteiles kann mittels eines in die Software des eingesetzten Computers aufgenommenen Rasters erfolgen, anhand dessen die für die Begrenzung der Endoprothese wichtigen Konturenpunkte festgelegt werden. Das Raster kann beispielsweise ein das einzelne Bereichsbild abdeckendes Gitterraster sein. Fig. 14, die im wesentlichen der Fig. 6 entspricht, zeigt ein Sternraster, das dort von einem in der Antetorsionsebene 23 liegenden Sternpunkt 43 ausgeht. Dieser Sternpunkt kann auch beispielsweise auf der in Fig. 1 eingezeichneten Seele 16 a liegen. Die Achse, auf welcher die Sternpunkte 43 aufeinanderfolgender Bereichsbilder liegen, kann also gerade, gekrümmt oder auch torquiert verlaufen. Auf den einzelnen Sternlinien 46 des Rasters wird anhand der Dichtewerte entweder der Beginn 47 der Übergangszone 44 zwischen Compacta 20 und Spongiosa 21 oder der Beginn 48 der Compacta 20 (starker Dichtewertwechsel) erfaßt. Von diesen ermittelten Punkten 47 oder 48 können automatisch z. B. strecken- oder winkelabhängige Korrekturabzüge vorgenommen werden, und anhand dieser korrigierten Werte wird dann die Kontur 49 der Endoprothese 16 (17) unter Beachtung der weiteren vorstehend genannten Verfahrensschritte festgelegt.

## Patentansprüche

1. Gelenkendoprothese (16) mit einem zum Einsetzen in einen von einer Knochenöffnung (12, 32) aus geschaffenen Knochenhohlraum (13) bestimmten, in einzelne Abschnitte (17) unterschiedlichen Querschnittes unterteilten, einen geradlinigen Bereich aufweisenden Verankerungsschaft (14, 14'), dadurch gekennzeichnet, daß der Verankerungsschaft (14, 14') mindestens in den Schaftbereichen (X, Y), in welchen eine Abstützung des Verankerungsschaftes (14, 14') am Knochen (10, 30) erfolgt, in einzelne, jeweils rundum verlaufende Stufen (17, 17', 17'') gegliedert ist, die mindestens in dem geradlinigen Bereich des Vekrankerungsschaftes von seinem gelenkfernen Ende (18) aus ohne Hinterschneidungen aufeinanderfolgen und einen sich trichterartig erweiternden Verankerungsschaft (14, 14') ergeben und deren Kontur mindestens über den Umfangsbereich, in welchem eine Abstützung der Stufen am Knochen (10, 30) erfolgt, individuell an denjenigen Querschnitt des Knochenhohlraumes (13) angepaßt ist, der bei eingesetzter Endoprothese (16) an der Stelle der einzelnen Stufen (17, 17', 17'') vorliegt, wobei die individuelle Anpassung durch folgende Schritte bewirkt worden ist:

a) Herstellung mehrerer maßstabsgerechter, nach den Prothesenschaftstufen (17 17' 17'') ausgewählter Bereichsbilder von dem mit der Endoprothese (16) zu versehenden Knochen (10, 30) mittels der Computer-Tomographie oder Kernspin-Resonanz-Tomographie;

b) Erfassung des von der Endoprothese (16) einzunehmenden Bereiches in den einzelnen Abbildungen und Speicherung dieser Bereichsdaten;

c) räumliche Kombination der Bildbereiche anhand der gespeicherten Bereichsdaten;

d) Überprüfung und Modifikation der Werte der räumlichen Kombination nach vorgegebenen Kriterien, wie Querschnittsverjüngung in einer vorgegebenen Richtung, Oberflächenreliefbildung, Hohlraumbildung, Belastbarkeit, Materialkonstanten;

e) Herstellung der Endoprothese (16) anhand der überprüften und modifizierten gespeicherten Kombinationswerte.

2. Endoprothese nach Anspruch 1, dadurch gekennzeichnet, daß der Verankerungsschaft (14, 14') sich im Bereich seines gelenknahen Endes stärker trichterartig erweitert als in seinen gelenkfernen Bereichen.

3. Endoprothese nach Anspruch 1 oder 2 als Hüftendoprothese, dadurch gekennzeichnet, daß ihr Verankerungsschaft (14) auf eine sogenannte Antetorsionsebene (23, 23', 23''), die nach dem Antetorsionswinkel des Gelenkkopfhalsanteils des zu ersetzenden Knochens gewählt wird, ausgerichtet aufgebaut ist.

4. Endoprothese nach Anspruch 3, dadurch gekennzeichnet, daß die sogenannte Antetorsionsebene (23', 23'') gekrümmt verläuft und/oder eine begrenzte Verwindung aufweist.

5. Endoprothese nach einem der Ansprüche 1 bis 4, dadurch gekennzeichnet, daß die Kanten (22) der Stufen (17') des Verankerungsschaftes gebrochen sind.

6. Endoprothese nach einem der Ansprüche 1 bis 5, dadurch gekennzeichnet, daß die Stufen (17) des Verankerungsschaftes (14, 14') in den bevorzugten Abstützbereichen (X, Y) und/oder den gekrümmten Bereichen des Schaftes (14) eine kleinere Höhe als in den übrigen Schaftbereichen aufweisen.

7. Endoprothese nach einem der Ansprüche 1 bis 6, dadurch gekennzeichnet, daß der gestufte Verankerungsschaft (14') an seinem gelenknahen Ende eine die Knochenöffnung (32) abdeckende Platte (33) trägt, die einen den Rand (35) der die Knochenöffnung (32) enthaltenden Knochenschnittfläche (31) umschließenden und an den Randverlauf angepaßten Randflansch (34) aufweist.

8. Endoprothese nach einem der Ansprüche 1 bis 7, dadurch gekennzeichnet, daß sie mit ihrem Verankerungsschaft (14, 14') aus einem gegossenen, geschmiedeten oder gepreßten Rohling (40) mittels materialabtragender Werkzeuge geformt ist.

9. Endoprothese nach Anspruch 8, dadurch gekennzeichnet, daß ihr Rohling (40) an beiden Enden nachträglich entfernbare, Einspann- oder Zentrierflächen bildende Ansatzkörper (41) aufweist.

10. Verfahren zur Herstellung einer individuellen mehrdimensionalen Gelenkendoprothese nach einem der Ansprüche 1 bis 9, dadurch gekennzeichnet, daß auf die ermittelten Bereichsbilder des Knochens(10, 30) ein Sternraster mit dem Sternpunkt (45) auf einer Bezugsachse (16a) liegend aufgebracht und auf den einzelnen Sternlinien (46) die Punkte (47, 48) festgelegt werden, die miteinander verbunden die Kontur (49) der diesem Bereich zugeordneten Endoprothesenstufe (17) ergeben.

**Claims**

1. A joint endoprosthesis (16) having an anchorage shaft (14, 14') which is intended for insertion into a bone cavity (13) created from a bone opening (12, 32), is subdivided into individual portions (17) of different cross-section, and has a rectilinear region, characterised in that the anchorage shaft (14, 14') is divided into individual steps (17, 17', 17''), at least in the shaft regions (X, Y) in which a supporting of the anchorage shaft (14, 14') against the bone (10, 30) is effected, which in each case extend all round and which at least in the rectilinear region of the anchorage shaft from its end (18) remote from the joint succeed one another without undercuts and result in the anchorage shaft (14, 14') widening in a funnel-like manner and the contour thereof adapted, at least over the circumferential region in which a supporting of the steps against the bone (10, 30) is effected, individually to that cross-section of the bone cavity (13) which, with the endoprosthesis (16) inserted, is present at the location of the individual steps (17, 17', 17''), in which respect the individual adaptation has been brought about by the following steps:

a) production of several true-to-scale regional images, selected in accordance with the prosthesis shaft steps (17, 17', 17''), of the bone (10, 30) that is to be provided with the endorprosthesis by means of computer tomography or nuclear-spin resonance tomography;

b) determination of the region, to be occupied by the endorposthesis (16), in the individual representations and storage of this regional data;

c) spatial combination of the image regions with the aid of the stored regional data;

d) checking and modification of the values of the spatial combination in accordance with predetermined criteria, auch as cross-sectional reduction in a predetermined direction, surface relief formation, cavity formation, loadability, material constants;

e) production of the endorprosthesis (16) with the aid of the checked and modified stored combination values.

2. An endoprosthesis according to claim 1, characterised in that the anchorage shaft (14, 14'), in the region of its end close to the joint, widens more severely in a funnel-like manner than in its regions remote from the joint.

3. An endoprosthesis according to claim 1 or 2 as a hip endoprosthesis, characterised in that its anchorage shaft (14) is constructed aligned at a so-called antetorsion plane (23, 23', 23'') which is selected in accordance with the antetorsion angle of the jointhead neck portion of the bone that is to be replaced.

4. An endoprosthesis according to claim 3, characterised in that the so-called antetorsion plane (23', 23'') extends in a curved manner and/or has a limited twist.

5. An endoprosthesis according to one of claims 1 to 4, characterised in that the edges (22) of the steps (17') of the anchorage shaft are chamfered.

6. An endoprosthesis according to one of claims 1 to 5, characterised in that the steps (17) of the anchorage shaft (14, 14') in the preferred supporting shaft regions (X, Y) and/or the curved regions of the shaft (14) have a smaller height than in the remaining shaft regions.

7. An endoprosthesis according to one of claims 1 to 6, characterised in that the stepped anchorage shaft (14') at its end adjacent the joint carries a plate (33) which covers the bone opening (32) and which has an edge flange (34) which surrounds the edge (35) of the bone sectional area (31) containing the bone opening (32) and which is adapted to the edge course.

8. An endoprosthesis according to one of claims 1 to 7, characterised in that with its anchorage shaft (14, 14') it is formed from a cast, forged or pressed blank (40) by means of material-removing tools.

9. An endoprosthesis according to claim 8, characterised in that its blank (40) has at both ends subsequently removable attachment bodies (41) which forms clamping or centring surfaces.

10. A method of producing an individual multidimensional joint endoprosthesis according to one of claims 1 to 9, characterised in that a star raster is applied, with the star point (45) lying on a reference axis (16a), to the determined regional images of the bone (10, 30) and on the individual star lines (46) the points (47, 48) are determined which, connected together, result in the contour (49) of the endoprosthesis step (17) associated with this region.

**Revendications**

1. Endoprothèse articulaire (16) munie d'une tige de fixation (14, 14') pour être implantée dans une cavité osseuse réalisée à partir d'une ouverture osseuse (12, 32), qui est subdivisée en plusieurs segments (17) de section transversale différente et qui comprend une portion rectiligne, caractérisée en ce que la tige de fixation (14, 14') est divisée au moins dans les zones du canal médullaire (X, Y) dans lesquelles la tige (14, 14') s'appuie sur l'os (10, 30), en plusieurs étages (17, 17', 17") disposés sur toute la périphérie, qui se suivent sans chevauchements tout au moins dans la portion rectiligne de la tige de fixation à partir de son extrémité (18) éloignée de l'articulation et forment une tige de fixation (14, 14') s'élargissant en entonnoir, et dont le contour est adapté, tout au moins sur la zone de la périphérie dans laquelle les étages s'appuient sur l'os (10, 30), individuellement à chaque section transversale de la cavité osseuse (13) qui existe à l'emplacement des différents étages (17, 17', 17") lorsque l'endoprothèse (16) est implantée, l'adaptation individuelle étant réalisée par les étapes suivantes:

a) Réalisation de plusieurs clichés de la région de l'os (10, 30) à munir de l'endoprothèse (16), en grandeur réelle, choisis en fonction des étages (17, 17', 17") de la tige de la prothèse, par tomographie sur ordinateur ou tomographie par résonance magnétique nucléaire;

b) Saise de la région d'implantation de l'endoprothèse (16) sur les différents clichés et mémorisation de ces données de région;

c) Combinaison spatiale des régions apparaissant sur les cliclés au moyen des données mémorisées;

d) Vérification et modificacion des valeurs de la combinaison spatiale d'après des critères prédéterminés, tels que rétrécissement de section transversale dans une direction prédéterminée, formation de relief superficiel, formation de cavité, capacité d'effort, constantes propres aux matières;

e) Fabrication de l'endoprothèse (16) d'après les valeurs de la combinaison mémorisées après vérification et modification.

2. Endoprothèse conforme à la revendication 1, caractérisée en ce que la tige de fixation (14, 14') s'élargit plus fortement en forme d'entonnoir dans la zone proximale à l'articulation que dans sa zone éloignée de l'articulation.

3. Endoprothèse conforme à l'une des revendications 1 ou 2, en tant qu'endoprothèse de la hanche, caractérisée en ce que en ce que sa tige de fixation (14) est montée en étant dirigée sur un plan dit d'antetorsion (23, 23', 23"), qui est sélectionné d'après l'angle d'antetorsion de la partie articulaire formée par la tête et le col de l'os à remplacer.

4. Endoprothèse conforme à la revendication 3, caractérisée en ce que le plan dit d'antetorsion (23', 23") est courbe et/ou présente une torsion limitée.

5. Endoprothèse conforme à l'une des revendications 1 à 4, caractérisée en ce que les bords (22) des étages (17') de la tige de fixation sont coupés.

6. Endoprothèse conforme à l'une des revendications 1 à 5, caractérisée en ce que les étages (17) de la tige de fixation (14, 14') présentent dans les zones d'appuis préférentielles (X, Y) et/ou dans les zones courbes de la tige de fixation (14) une hauteur inférieure à celle des autres zones de la tige.

7. Endoprothèse conforme à l'une des revendications 1 à 6, caractérisée en ce que la tige de fixation étagée (14') porte à son extrémité proche de l'articulation une plaque (33) recouvrant l'ouverture osseuse (32), qui présente une bride marginale (34) entourant la surface coupée de l'os (31) qui comprend le bord (35) de l'ouverture osseuse (32), et adaptée à la forme du bord.

8. Endoprothèse conforme à l'une des revendications 1 à 7, caractérisée en ce qu'elle est formée avec sa tige de fixation (14, 14') à partir d'une ébauche coulée, forgée ou matricée (40) au moyen d'outils enlevant de la matière.

9. Endoprothèse conforme conforme à la revendication 8, caractérisée en ce que son ébauche (40) comporte à ses deux extrémités des corps ajoutés (41), amovibles ultérieurement, formant des surfaces de serrage ou de centrage.

10. Procédé de fabrication d'une endoprothèse articulaire individuelle à plusieurs dimensions conforme à l'une des revendications 1 à 9, caractérisé en ce qu'une trame en étoile, dont le point d'origine (45) est situé sur un axe de référence (16a), est disposée sur les tomogrammes de l'os (10, 30) et que sur les différentes lignes en étoile (46) sont déterminés les points (47, 48) qui, liés entre eux, donnent le contour (49) de l'étage (17) de l'endoprothèse affecté à cette zone.

FIG.1

FIG.2

FIG.3

FIG. 4

FIG.5

FIG.6

FIG.7

17

14

23

23'

FIG.9

23"

FIG.8

FIG.10

7

FIG.11

FIG.12

FIG.13

0 093 869

FIG.14